(19) Europäisches Patentamt
European Patent Office
Office européen des brevets

(11) Numéro de publication: **0 002 635**
**B1**

(12) **FASCICULE DE BREVET EUROPEEN**

(21) Numéro de dépôt: **78400210.7**

(22) Date de dépôt: **04.12.78**

(51) Int. Cl.³: **C 07 D 495/04**,
A 61 K 31/44,
(C 07 D 495/04, 333/00,
221/00)

# (54) Procédé de préparation de dérivés de thiéno (2,3-c) et (3, 2-c) pyridines, nouveaux dérivés de la thiéno (2,3-c) pyridine obtenus et leur application thérapeutique

(30) Priorité: **19.12.77 FR 7738308**

(43) Date de publication de la demande:
**27.06.79 Bulletin 79/13**

(45) Mention de la délivrance du brevet:
**20.08.80 Bulletin 80/17**

(84) Etats contractants désignés:
**DE NL SE**

(56) Documents cités:
**Néant**

(73) Titulaire: **PARCOR**
**40, avenue Georges V**
**F - 75008 Paris (FR)**

(72) Inventeurs: **Frehel, Daniel**
**Résidence La Pléiade 28 Boulevard de la Marne**
**F - 31400 Toulouse (FR)**
**Maffrand, Jean-Pierre**
**10 Rue Jean Mermoz**
**F - 31300 Toulouse (FR)**

(74) Mandataire: **Lavoix, Jean**
**c/o Cabinet Lavoix 2, Place D'Estienne D'Orves**
**F - 75441 Paris Cedex 09 (FR)**

EP 0 002 635 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de dérivés de thiéno [2,3-c] et [3,2-c] pyridines, nouveaux dérivés de la thiéno [2,3-c] pyridine obtenus et leur application thérapeutique.-

La présente invention est relative à un nouveau procédé de préparation de dérivés de la thiéno pyridine, aux nouveaux composés ainsi obtenus et à leur application en médecine humaine et vétérinaire.

Ces nouveaux dérivés peuvent aussi être employés comme intermédiaires dans la synthèse de composés utilisables aussi bien dans l'industrie chimique que pharmaceutique.

On connaît déjà d'après le brevet US 3 845 065 des dérivés de thiéno [3,2-c] pyridine non substitués en position 4, leur application thérapeutique, notamment comme anti-inflammatoires, ainsi que des procédés pour leur préparation.

L'invention a pour objet un nouveau procédé de préparation de dérivés de thiéno [2,3-c] et [3,2-c] pyridines de formules respectives suivantes:

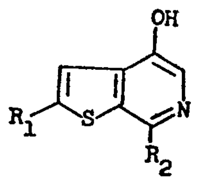
(I) et
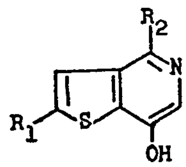
(II)

dans lesquelles $R_1$ et $R_2$ sont identiques ou différents et représentent chacun un atome d'hydrogène ou un groupe alcoyle, et leurs sels d'addition avec des acides pharemaceutiquement acceptables, caractérisé en ce que:

a) on condense un composé de formule VII ou VIII suivante:

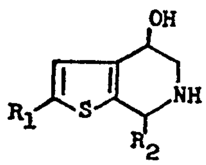
(VII) ou
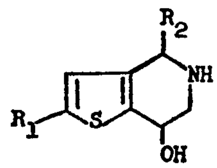
(VIII)

avec un chlorure de sulfonyle de formule $ClSO_2R_3$, dans laquelle $R_3$ est un radical alcoyle inférieur ou un radical phényle éventuellement substitué par un atome d'halogène ou un groupe alcoyle inférieur, dans un système solvant biphasique et en présence de carbonate de sodium;

b) on oxyde dans un milieu solvant les alcools obtenus de formule V ou VI suivante:

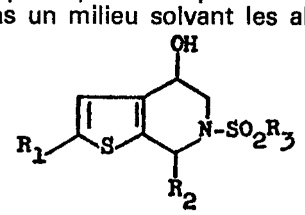
ou
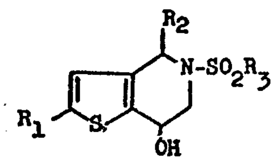

(V) (VI)

c) on traite les cétones obtenues de formule III ou IV suivante:

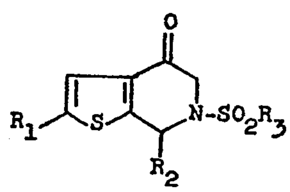
ou
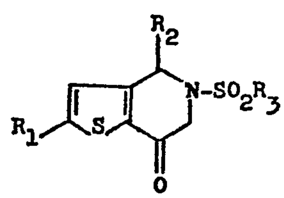

(III) (IV)

par un agent basique de formule $RO^- M^+$, dans lequel R est un radical alcoyle aliphatique ramifié ou non et $M^+$ est un cation d'un métal alcalin, dans un alcool de formule ROH comme solvant et à la température de reflux du mélange réactonnel, pour obtenir respectivement les composés de formule I ou II.

Les formules générales I et II peuvent également s'écrire sous leur forme zwitterion:

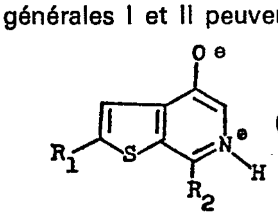
(I') et
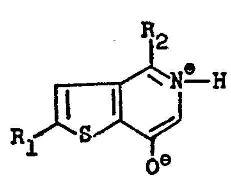
(II')

Les groupes $R_1$ et $R_2$ sont notamment des groupes alcoyle inférieurs ayant de 1 à 6 atomes de carbone tel que, par exemple méthyle.

Elle a aussi pour objet des dérivés de thiéno [2,3-c] pyridine de formule suivante:

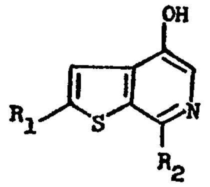

(I)

dans laquelle $R_1$ et $R_2$ sont identiques ou différents et représentent chacun un atome d'hydrogène ou un groupe alcoyle, et leurs sels d'addition avec des acides pharmaceutiquemant acceptables.

L'étape essentielle du procédé de préparation décrit ci-dessus réside dans le traitement par un agent basique d'un dérivé de formule (III) ou (IV) suivante:

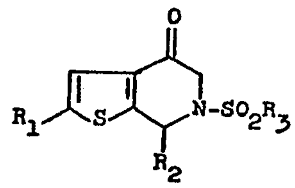

(III)

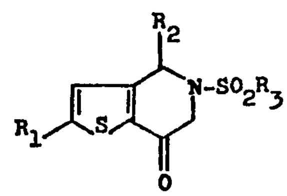

(IV)

dans lesquelles $R_1$ et $R_2$ ont les significations données précédemment et $R_3$ est un radical alcoyle inférieur, de préférence un groupe méthyle, ou un radical phényle éventuellement substitué par un atome d'halogène ou un groupe alcoyle inférieur tel que méthyle. La réaction consiste à éliminer un acide sulfinique sous la forme de son sel $R_3SO_2^-$ $M^+$, sous l'influence d'un alcoolate $RO^-$ $M^+$, selon le schéma:

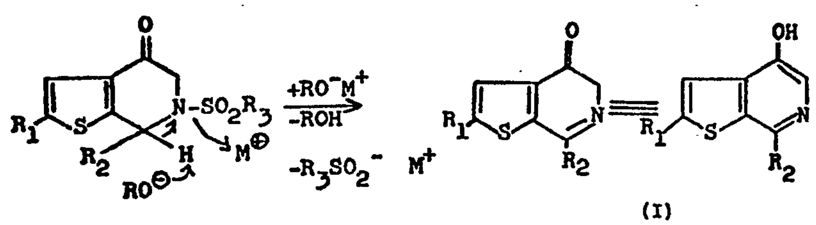


(I)

De même, par traitement du composé de formule (IV), on obtient le composé de formule II.

Cette réaction s'effectue par chauffage à reflux dans un alcool aliphatique ROH, en $C_1$ à $C_5$, ramifié ou non, en présence de son alcoolate de sodium ou de potassium ($RO^-$ $Na^+$ ou $RO^-$ $K^+$). L'utilisation de tertiobutylate de potassium dans le tertiobutanol est particulièrement avantageuse.

Les composés (III) et (IV) sont obtenus par oxydation des alcools (V) et (VI) correspondants:

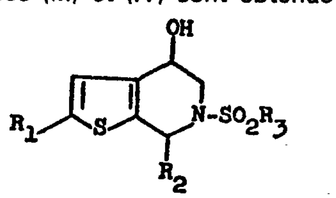

(V)

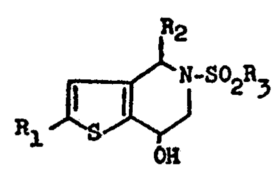

(VI)

La réaction est effectuée dans l'acétone en utilisant une solution d'anhydride chromique dans l'acide sulfurique comme agent oxydant.

Les alcools (V) et (VI) sont eux-mêmes préparés par condensation des tétrahydro-4,5,6,7 thiénopyridines correspondantes (VII) et (VIII) avec un chlorure de sulfonyle $ClSO_2R_3$.

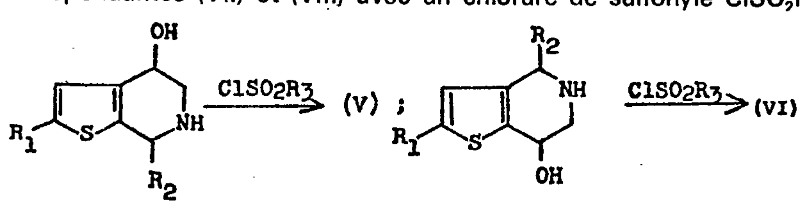


(VII) (VIII)

La réaction s'effectue dans le système biphasique eau-chloroforme en présence de carbonate de sodium ou de potassium.

Les dérivés (V) et (VI) pour lesquels $R_1 = R_2 = H$ et $R_3$ = p-tolyle ont déjà été mentionnés dans la littérature par J.P. Maffrand et F. Eloy, J. Het. Chem. 1976, 13, 1347.

Les produits de départ (VII) et (VIII) pour lesquels $R_1 = H$ sont décrits dans l'article ci-dessus ou dans les brevets français du titulaire No 2 278 337 et 2 313 055.

On donnera dans le présent document un exemple de préparation du dérivé (VII) avec $R_1 = CH_3$.

Les exemples suivants illustrent l'invention.

Préparation de l'hydroxy-4 méthyl-2 tétrahydro-4,5,6,7 thiéno [2,3-c] pyridine:(VII):$R_1 = CH_3$, $R_2 = H$.

Dans un ballon équipé d'un séparateur d'eau (Dean-Stark) surmonté d'un réfrigérant, on chauffe au reflux pendant 2 heures, un mélange de 30 g (0,237 mole) de méthyl-5- thiophène carboxaldéhyde-2, 27,4 g (0,261 mole) d'aminoacétaldéhyde diméthylacétal dans 250 cm3 de benzène. On évapore à sec et dissout le résidu dans 250 cm3 d'ethanol. On ajoute, par portions, 13,5 g (0,355 mole) de borohydrure de sodium et abandonne une nuit à température ambiante. L'excès de borohydrure de sodium est détruit par addition d'acétone et le mélange est évaporé à sec. Le résidu est repris par de l'eau et extrait au chlorure de méthylène. Les extraits organiques sont séchés sur sulfate de sodium, et évaporés à sec. L'huile résiduelle obtenue est distillée sous pression réduite, $Eb_2$ = 127°C, rendement: 90%. Le N-(méthyl-5- thiényl-2) méthyl aminoacétaldéhyde diméthylacétal ainsi obtenu est chauffé à 60°C, pendant 1 heure, dans 250 cm3 d'acide chlorhydrique 6 N.

On évapore à sec et triture le résidu avec de l'acétone. Les cristaux blanchâtres du chlorhydrate recherché sont recristallisés dans l'acétonitrile:F = 120°C, rendement global:61%.

Exemple 1

Préparation de l'hydroxy-7 thiéno [3,2-c] pyridine:dérivé 1 (II):$R_1 = R_2 = H$

a) Préparation de l'hydroxy-7 tosyl-5 tétrahydro-4,5,6,7 thiéno [3,2-c] pyridine. (VI):$R_1 = R_2 = H$; $R_3$ = p-tolyle

A un mélange de 45 g (0,234 mole) de chlorhydrate d'hydroxy-7 tétrahydro-4,5,6,7 thiéno [3,2-c] pyridine, 100 cm3 de chloroforme et 150 cm3 d'une solution aqueuse saturée de carbonate de potassium, on ajoute, goutte à goutte, à température ambiante et sous agitation mécanique vigoureuse, une solution de 45 g (0,234 mole) de chlorure de p-toluènesulfonyle dans 250 cm3 de chloroforme et on poursuit l'agitation pendant 4 Heures. Après décantation, la phase chloroformique est lavée à l'eau, séchée sur sulfate de sodium anhydre et évaporée à sec. Le résidu est purifié par chromatographie sur colonne de silice (éluant: toluène-acétate d'éthyle 7—3) et recristallisation dans l'isopropanol.

Cristaux blancs, F = 120°C, rendement:74%.

b) Préparation de l'oxo-7 tosyl-5 tétrahydro-4,5,6,7 thiéno [3,2-c] pyridine (IV):$R_1 = R_2 = H$; $R_3$ = p-tolyle

On ajoute, goutte à goutte, sous bonne agitation et à température ambiante, 28,4 cm3 de réactif de Jones (solution 2,50 M d'anhydride chromique dans de l'acide sulfurique 8,35 N à une solution de 20,3 g (0,064 mole) d'hydroxy-7 tosyl-5 tétrahydro-4,5,6,7 thiéno [3,2-c] pyridine préparée sous a), dans 250 cm3 d'acétone. On poursuit l'agitation à température ambiante pendant 2 heures, filtre les sels minéraux précipités, évapore à sec le filtrat et reprend le résidu par du chlorure de méthylène. La phase organique est lavée avec une solution aqueuse à 5% de bicarbonate de sodium, puis à l'eau, séchée sur sulfate de sodium et évaporée à sec. Le résidu solide est recristallisé dans le benzène.

Cristaux crème, F = 174°C, rendement:79%.

c) Préparation de l'hydroxy-7 thiéno [3,2-c] pyridine (II):$R_1 = R_2 = H$, dérivé 1

On chauffe à reflux pendant 2 heures, sous atmosphère d'azote, une solution de 19,6 g (0,064 mole) d'oxo-7 tosyl-5 tétrahydro-4,5,6,7 thiéno [3,2-c] pyridine ((b) ci-dessus), 28,6 g (0,255 mole) de tertio-butylate de potassium dans 300 cm3 de tertiobutanol. L'évaporation sous vide du solvant laisse un résidu que l'on reprend par de l'acide chlorhydrique 2N. La phase aqueuse est extraite à éther, puis basifiée par addition d'ammoniaque concentrée et évaporée à sec. Le résidu est extrait quatre fois avec de l'acétate d'éthyle bouillant. Les extraits organiques sont filtrés sur lit de silice et évaporés à sec. Le résidu solide est recristallisé dans un mélange éthanol-acétonitrile.

Cristaux grisâtres, F = 180°C, rendement:72%.

Exemple 2

Préparation de l'hydroxy-4 thiéno [2,3-c] pyridine:dérivé 2, (I):$R_1 = R_2 = H$

a) Préparation de l'hydroxy-4 tosyl-6 tétrahydro-4,5,6,7 thiéno [2,3-c] pyridine. (V) $R_1 = R_2 = H$; $R_3$ = p-tolyle

Ce composé est préparé selon le mode opératoire décrit à l'exemple 1 a) à partir du chlorhydrate d'hydroxy-4 tétrahydro-4,5,6,7 thiéno [2,3-c] pyridine.

Cristaux beiges, F = 130°C (isopropanol), rendement:86%.

b) Préparation de l'oxo-4 tosyl-6 tétrahydro-4,5,6,7 thiéno [2,3-c] pyridine (III) $R_1 = R_2 = H$, $R_3$ = p-tolyle

Ce composé est préparé selon le mode opératoire décrit à l'exemple 1 b) à partir du tosylate décrit sous a) ci-dessus.

Cristaux blancs translucides, F = 172°C (benzène), rendement:98%.

c) Préparation de l'hydroxy-4 thiéno [2,3-c] pyridine (I):$R_1 = R_2 = H$, dérivé 2.

Ce composé est préparé selon le mode opératoire décrit à l'exemple 1 c) à partir du tosylate décrit sous b) ci-dessus (rendement:78%).

Cristaux blancs, F = 206°C (éthanol-cyclohexane).

Exemple 3

Préparation de l'hydroxy-4 méthyl-7 thiéno [2,3-c] pyridine:(I) dérivé 3, $R_1$ = H, $R_2$ = $CH_3$

a) Préparation de l'hydroxy-4 méthyl-7 tosyl-6 tétrahydro-4,5,6,7 thiéno [2,3-c] pyridine. (V):$R_1$ = H, $R_2$ = $CH_3$, $R_3$ = p-tolyle

Ce composé est préparé selon le mode opératoire décrit à l'exemple 1 a) à partir du chlorhydrate d'hydroxy-4 méthyl-7 tétrahydro-4,5,6,7 thiéno [2,3-c] pyridine.

Cristaux blanchâtres, F = 120°C (benzène-cyclohexane), rendement:96%.

b) Préparation de la méthyl-7 oxo-4 tosyl-6 tétrahydro-4, 5,6,7 thiéno [2,3-c] pyridine. (III) = $R_1$ = H, $R_2$ = $CH_3$, $R_3$ = p-tolyle

Ce composé est préparé selon le mode opératoire décrit à l'exemple 1 b) à partir du tosylate décrit sous a) ci-dessus.

Cristaux blancs, F = 164°C (benzène-acétone), rendement:90%.

c) Préparation de l'hydroxy-4 méthyl-7 thiéno [2,3-c] pyridine (I):$R_1$ = H, $R_2$ = $CH_3$, dérivé 3.

Ce composé est préparé selon le mode opératoire décrit à l'exemple 1 c) à partir du tosylate décrit sous b) ci-dessus.

Cristaux blancs, F = 220°C (cyclohexane-éthanol), rendement:50%.

Exemple 4

Préparation de l'hydroxy-4 méthyl-2 thiéno [2,3-c] pyridine (I) dérivé 4, $R_1$ = $CH_3$, $R_2$ = H

a) Préparation de l'hydroxy-4 méthyl-2 tosyl-6 tétrahydro-4,5,6,7 thiéno [2,3-c] pyridine (V):$R_1$ = $CH_3$, $R_2$ = H, $R_3$ = p-tolyle.

Ce composé est préparé selon le mode opératoire décrit à l'exemple 1 a) à partir du chlorhydrate d'hydroxy-4 méthyl-2 tétrahydro-4,5,6,7 thiéno [2,3-c] pyridine (préparation préliminaire).

Cristaux blancs, F = 132°C (benzene), rendement:48%.

b) Préparation de la méthyl-2 oxo-4 tosyl-6 tétrahydro-4,5,6,7 thiéno [2,3-c] pyridine (III):$R_1$ = $CH_3$, $R_2$ = H, $R_3$ = p-tolyle.

Ce composé est prépare selon le mode opératoire décrit à l'exemple 1 (b) à partir du tosylate décrit sous a) ci-dessus.

Cristaux blanchâtres, F = 124°C, rendement:83%.

c) Préparation de l'hydroxy-4 méthyl-2 thiéno [2,3-c] pyridine (I):$R_1$ = $CH_3$, $R_2$ = H, dérivé 4.

Ce composé est préparé selon le mode opératoire décrit à l'exemple 1 c) à partir du tosylate décrit sous b) ci-dessus.

Cristaux grisâtres, F = 220°C (éthanol-acétonitrile), rendement:36%.

Exemple 5

Préparation de l'hydroxy-4 thiéno [2,3-c] pyridine:dérivé 2

Cet exemple est une variante du procédé de préparation du dérivé 2 illustré à l'exemple 2.

a) Préparation de l'hydroxy-4-mésyl-6 tétrahydro-4,5,6,7 thiéno [2,3-c] pyridine. (V):$R_1$ = $R_2$ = H, $R_3$ = $CH_3$

A un mélange de 50 g (0,26 mole) de chlorhydrate d'hydroxy-4 tétrahydro-4,5,6,7 thiéno [2,3-c] pyridine, 200 cm3 de chloroforme et 100 cm3 d'une solution aqueuse saturée de carbonate de potassium, on ajoute, goutte à goutte, à température ambiante et sous agitation mécanique vigoureuse une solution de 30 g (0,26 mole) de chlorure de méthanesulfonyle dans 50 cm3 de chloroforme. On poursuit l'agitation pendant 2 heures, à température ambiante. Après décantation la phase chloroformique est lavée avec de l'acide chlorhydrique dilué, puis à l'eau et séchée sur sulfate de sodium anhydre. L'évaporation sous vide du solvant laisse des cristaux que l'on recristallise dans le méthanol.

Cristaux brun clain, F = 140°C (méthanol), rendement:76%.

b) Préparation de la mésyl-6 oxo-4 tétrahydro-4,5,6,7 thiéno [2,3-c] pyridine (III):$R_1$ = $R_2$ = H, $R_3$ = $CH_3$

Ce composé est préparé selon le mode opératoire décrit à l'exemple 1 b) à partir du méthanesulfonate décrit sous a) ci-dessus.

Cristaux beiges, F = 120°C (isopropanol-acétate d'éthyle), rendement:70%.

c) Préparation de l'hydroxy-4 thiéno [2,3-c] pyridine (I) $R_1$ = $R_2$ = H

Ce composé est préparé selon le mode opératoire décrit à l'exemple 1 c) à partir du méthanesulfonate décrit sous b) ci-dessus (rendement:80%).

Cristaux blancs, F = 206°C (éthanol-cyclohexane).

Les résultats des essais toxicologiques et pharmacologiques qui sont rapportés ci-après, ont mis en évidence les intéressantes activites des dérivés de formules (I) et/ou (II), notamment anti-inflammatoires.

L'invention a encore pour objet un médicament présentant notamment des activites anti-inflammatoires, caractérisé en ce qu'il contient, à titre de principe actif, une quantité efficace d'un dérivé de formule (I) et/ou d'un sel d'addition avec un acide pharmaceutiquement acceptable de ce dérivé, en association avec des véhicules et excipients usuels.

I — Etude toxicologique

Les composés de l'invention bénéficient d'une excellente tolérance et d'une faible toxicité. Ainsi, la DL 50/ 24 H/kg de poids corporel d'animal, déterminée chez la souris selon la méthode de Miller et Tainter, pour la voie orale, est supérieure à 800 mg pour tous les dérivés.

Pour la voie intraveineuse, à titre d'exemple, la DL 50 déterminée chez la souris a été de 131 mg pour le dérivé 1.

En outre, les essais effectués sur la toxicité aigüe, chronique, sub-chronique et retardée, chez diverses espèces animales, n'ont mis en évidence aucune réaction locale ou générale, aucune perturbation dans les contrôles biologiques régulièrement effectués, aucune anomalie dans les examens microscopiques et macroscopiques chez les animaux sacrifiés et autopsiés en fin d'expérimentation.

II — Etude pharmacologique

L'étude pharmacologique a porté sur l'action anti-inflammatoire des composés de l'invention. Cette action a été étudiée selon 2 méthodes:

a) Méthode de l'oedème localisé provoqué par la carragénine.

Une solution de carragénine à 1% (0,1 ml) est injectée dans les fléchisseurs métatarsiens de la patte postérieure droite du rat au temps 0. Les animaux du lot traité reçoivent en outre, par la voie orale, 100 mg par kg de poids corporel du dérivé à tester, respectivement 1 heure avant, en même temps que l'injection de l'agent phlogogène, puis 1 heure et 2 heures et demie après. Les mesures qui sont effectuées à l'aide du micromètre de ROCH aux temps 0, 1 heure, 2 heures, 3 heures et 5 heures après l'administration de la carragénine permetent de déterminer, en fonction du temps, le pourcentage d'activité anti-inflammatoire, par comparaison avec le lot témoin.

Les résultats sont consignés dans le Tableau I suivant:

TABLEAU I

Pourcentage d'activité anti-inflammatoire

| Dérivé No. | 1ère heure | 2ème heure | 3ème heure | 5ème heure |
|---|---|---|---|---|
| 1 | 43 | 46 | 48 | 49 |
| 2 | 40 | 45 | 49 | 49 |
| 3 | 42 | 48 | 52 | 51 |
| 4 | 45 | 49 | 50 | 52 |

b) Méthode de l'oedème généralisé à l'ovalbumine

Une injection intrapéritonéale simultanée de 1 ml d'ovalbumine et de 0,5 ml d'une solution aqueuse de bleu Evans à 1% est effectuée sur le rat. D'autre part, on administre per os aux animaux du lot traité 100 mg/kg de poids corporel du dérivé à tester 1 heure avant et en même temps que l'ovalbumine. L'intensité du phénomène ainsi provoqué est notée par un chiffre allant de 1 à 5 suivant la progression du syndrome inflammatoire. On détermine ainsi la moyenne de l'intensité oedémateuse et le pourcentage de diminution de la réaction oedémateuse par rapport au témoin, en fonction du temps.

Les pourcentages d'activité anti-inflammatoire obtenue à la 2ème heure et 3ème heure après l'injection d'ovalbumine sont consignés dans le Tableau II suivant:

TABLEAU II

Pourcentage d'activite anti-inflammatoire

| Dérivé No. | 2ème heure | 3ème heure |
|---|---|---|
| 1 | 44 | 50 |
| 2 | 49 | 58 |
| 3 | 53 | 61 |
| 4 | 48 | 60 |

Les résultats de ces études mettent en évidence la faible toxicité et les intéressantes proprietes anti-inflammatoires des dérivés de l'invention qui les rendent très utiles en médecine humaine et vétérinaire.

Le médicament de l'invention peut être présenté, pour l'administration orale, sous forme de comprimés, comprimés dragéifiés, capsules, gouttes et sirop. Il peut aussi être présenté, pour l'administration rectale, sous forme de suppositoires et pour l'administration parentérale sous forme de soluté injectable.

Chaque dose unitaire contient avantageusement de 0,010 g à 0,250 g de principe actif, les doses administrables journellement pouvant varier de 0,010 g à 0,750 g de principe actif selon l'âge du patient et l'affection traitée.

On donnera ci-après, à titre d'exemples non limitatifs, quelques formulations pharmaceutiques du médicament de l'invention:

1°-Comprimés

| | |
|---|---|
| dérivé No 2 | 0,100 g |

excipient: amidon de blé, lactose, gomme arabique, silice, stéarate de magnésium.

2°-Comprimés dragéifiés

| | |
|---|---|
| dérivé No 4 | 0,075 g |

excipient: talc, polyvinylpyrrolidone, stéarate de magnésium, carbonate de calcium, gomme laque, gomme arabique, sucre, oxyde de titane, glucose, cire blanche, colophane, cire de carnauba, lactose, saccharose, jaune tartrazine, bleu patenté.

3°-Capsules

| | |
|---|---|
| dérivé No 3 | 0,150 g |

excipient: talc, amidon de maïs, acide stéarique

4°-Ampoules injectables

| | |
|---|---|
| dérivé No 2 | 0,100 g |

excipient: solvant isotonique q.s.p. 5 ml

5°-Suppositoires

| | |
|---|---|
| dérivé No 4 | 0,100 g |

excipient: triglycérides semi-synthétiques

Les études toxicologiques et pharmacologiques qui viennent d'être rapportées ont mis en évidence la bonne tolérance des dérivés de l'invention ainsi que leurs activités anti-inflammatoires importantes.

Le médicament de l'invention peut ainsi être administré à l'homme avec profit dans le traitement de tous les états inflammatoires, quelle que soit leur étiologie:rhumatismes inflammatoires chroniques, rhumatismes dégénératifs, affections ab-articulaires, affections inflammatoires de la sphère oto-rhino-laryngologique, en traumatologie, en odonto-stomatologie, en chirurgie post-opératoire et en gynécologie.

**Revendications**

1. Procédé de préparation de dérivés de thiéno [2,3-c] et [3,2-c] pyridines de formules respectives suivantes:

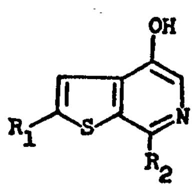 (I) et 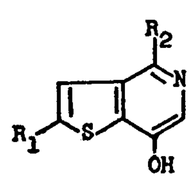 (II)

dans lesquelles $R_1$ et $R_2$ sont identiques ou différents et représentent chacun un atome d'hydrogène ou un groupe alcoyle, et leurs sels d'addition avec des acides pharmaceutiquement acceptables, caractérisé en ce que:

a) on condense un composé de formule VII ou VIII suivante:

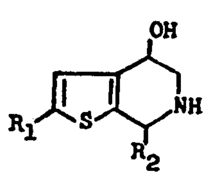 (VII) et 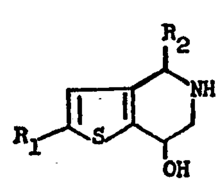 (VIII)

avec un chlorure de sulfonyle de formule $ClSO_2R_3$, dans laquelle $R_3$ est un radical alcoyle inférieur ou un radical phényle éventuellement substitué par un atome d'halogène ou un groupe alcoyle inférieur, dans un système solvant biphasique et en présence de carbonate de sodium;

b) on oxyde dans un milieu solvant les alcools obtenus de formule V ou VI suivante:

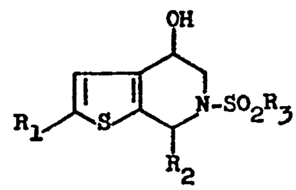

(V)

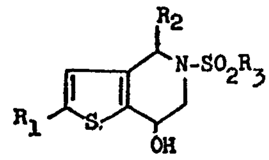

(VI)

c) on traite les cétones obtenues de formule III ou IV suivante:

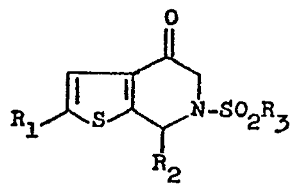

(III)

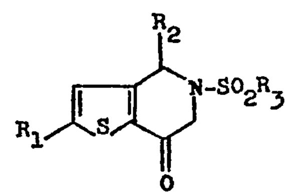

(IV)

par un agent basique de formule $RO^-M^+$, dans lequel R est un radical alcoyle aliphatique ramifié ou non et $M^+$ est un cation d'un métal alcalin, dans un alcool de formule ROH comme solvant et à la température de reflux du mélange réactionnel, pour obtenir respectivement les composés de formule I ou II.

2. Procédé selon la revendication 1, caractérisé en ce que le système biphasique de l'étape a) est un mélange eau-chloroforme.

3. Procédé selon la revendication 1, caractérisé en ce que l'agent oxydant utilisé à l'étape b) est une solution d'anhydride chromique dans l'acide sulfurique et le milieu solvant est l'acétone.

4. Procédé selon la revendication 1, caractérisé en ce que l'agent basique utilisé est un alcoolate de sodium ou de potassium en $C_1$ à $C_5$, tel que le tertiobutylate de potassium, et l'alcool R OH est l'alcool tertiobutylique.

5. Dérivés de thiéno [2,3-c] pyridine de formule suivante:

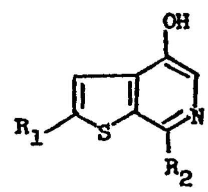

(I)

dans laquelle $R_1$ et $R_2$ sont identiques ou différents et représentent chacun un atome d'hydrogène ou un groupe alcoyle, et leurs sels d'addition avec des acides pharmaceutiquement acceptables.

6. Dérivés selon la revendication 5, caractérisés en ce que le radical alcoyle est un radical alcoyle inférieur ayant de 1 à 6 atomes de carbone.

7. Composition thérapeutique ayant notamment des activités anti-inflammatoires, caractérisée en ce qu'elle contient à titre de principe actif une quantité efficace d'au moins un composé selon la revendication 5, en association avec des véhicules et excipients pharmaceutiquement acceptables.

8. Composition thérapeutique selon la revendication 7, caractérisée ce qu'elle est présentée souse forme de doses unitaires contenant de 10 à 250 mg de principe actif.

**Patentsprüche**

1. Verfahren zur Herstellung von Derivaten von Thieno [2,3-c]-und Thieno [3,2-c]-pyridinen der folgenden entsprechenden Formeln:

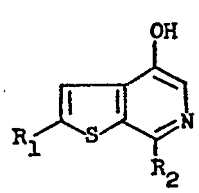

(I) und

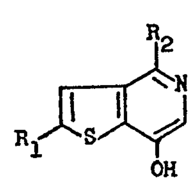

(II)

in denen $R_1$ und $R_2$ gleich oder verschieden sind, und für Wasserstoff oder Alkylgruppen stehen, sowie ihre Additionssalze mit pharmazeutisch verträglichen Säuren, dadurch gekennzeichnet,

a) daß man eine Verbindung der folgenden Formeln VII oder VIII

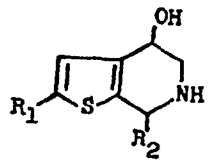 (VII) und 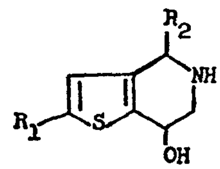 (VIII)

mit einem Sulfonylchlorid der Formel $ClSO_2R_3$, in der $R_3$ eine niedere Alkylgruppe oder eine gegebenenfalls mit Halogen oder einer niederen Alkylgruppe substituierte Phenylgruppe ist, in einem zweiphasigen Lösungsmittelsystem und in Gegenwart von Natriumcarbonat kondensiert;

b) daß man in einem Lösungsmittelsystem die gebildeten Alkohole der folgenden Formeln V oder VI oxidiert:

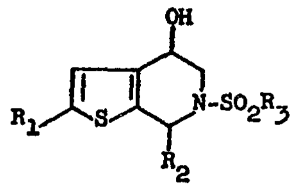

(V)

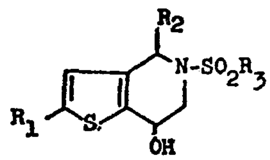

(VI)

c) daß man die gebildeten Ketone der folgenden Formeln III oder IV

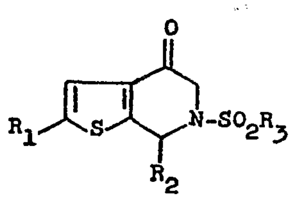

(III)

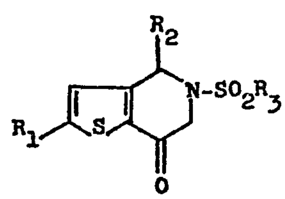

(IV)

mit einem basischen Mittel der Formel $RO^-M^+$, in der R eine aliphatische verzweigte oder unverzweigte Alkylgruppe und $M^+$ ein Alkalimetallkation sind, in einem Alkohol der Formel ROH als Lösungsmittel und bei Rückflußtemperatur der Reaktionsmischung zur bildung der Verbindungen der Formel I oder II behandelt.

2. Verfahren nach Anspruch 1, dadurch gekennzeichnet, daß das zweiphasige System in Stufe a) eine Wasser/Chloroformmischung ist.

3. Verfahren nach Anspruch 1, dadurch gekennzeichnet, daß man als oxidierendes Mittel in der Verfahrensstufe b) eine Lösung von Chromsäureanhydrid in Schwefelsäure und als Lösungsmittel Aceton verwendet.

4. Verfahren nach Anspruch 1, dadurch gekennzeichnet, daß man als basisches Mittel ein Natriumalkoholat oder ein Kaliumalkoholat mit 1 bis 5 Kohlenstoffatomen wie Kalium-tert, butylat und als Alkohol ROH tert, Butylalkohol verwendet.

5. Derivate von Tieno [2,3-c]-pyridin der folgenden Formel

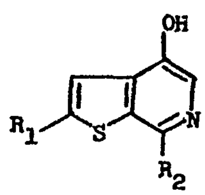 (I)

in der $R_1$ und $R_2$ gleich oder verschieden sind und für Wasserstoff oder eine Alkylgruppe stehen, sowie ihre Additionssalze mit pharmazeutisch verträglichen Säuren.

6. Derivate nach Anspruch 5, dadurch gekennzeichnet, daß die Alkylgruppe eine niedere Alkylgruppe mit 1 bis 6 Kohlenstoffatomen ist.

7. Therapeutische Zusammensetzung mit entzündungshemmender Wirkung, dadurch gekennzeichnet, daß sie als aktiven Wirkstoff in wirksamer Menge mindestens eine Verbindung gemäß Anspruch 5 zusammen mit pharmazeutisch verträglichen Trägerstoffen und Exzipientien enthält.

8. Therapeutische Zusammensetzung nach Anspruch 7, dadurch gekennzeichnet, daß sie in Form von Einheitsdosen mit einem Gehalt von 10 bis 250 mg an aktivem Wirkstoff zur Verfügung gestellt wird.

**Claims**

1. Process for the preparation of thieno [2,3-c]- and [3,2,-c] pyridines, having repectively the following formulae:

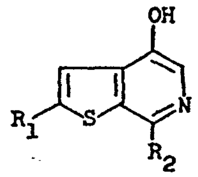 (I) and 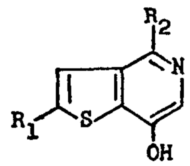 (II)

in which $R_1$ and $R_2$, which may be the same or different, represent each a hydrogen atom or an alkyl group, and their pharmaceutically acceptable acid addition salts, comprising:

a) condensing a compound of the following formula (VII) or (VIII), respectively,

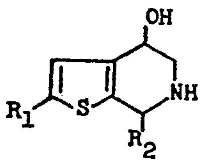 (VII) or 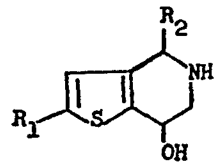 (VIII)

with a sulfonyl chloride having the formula $ClSO_2R_3$, in which $R_3$ is a lower alkyl radical or a phenyl radical optionally substituted with a halogen atom or a lower alkyl group, within a two-phase solvent system and in the presence of sodium carbonate;

b) oxidizing in a solvent medium the resulting alcohols having respectively the following formula (V) or (VI):

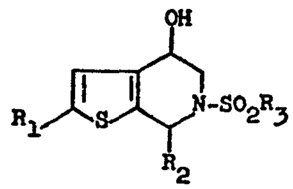 or 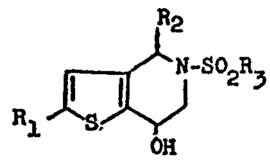

(V) (VI)

c) treating the resulting ketones, having the following formula (III) or (IV):

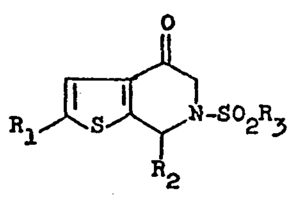 or 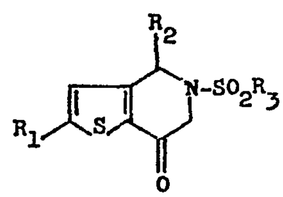

(III) (IV)

with a basic agent having the formula $RO^-M^+$ in which R is a branched- or straight-chain aliphatic alkyl radical and $M^+$ is an alkali metal cation, within an alcohol solvent having the formula ROH, and at the reflux temperature of the reaction mixture, to give the compounds of the formula (I) or (II), respectively.

2. Process as claimed in claim 1, wherein the two-phase solvent system of step a) is a water-chloroform mixture.

3. Process as claimed in claim 1, wherein the oxidizing agent used in step b) is a solution of chromic anhydride in sulfuric acid and the solvent medium is acetone.

4. Process as claimed in claim 1, wherein the basic agent used is a $C_{1-5}$ sodium or potassium alkoxide, such as potassium tert-butoxide, and the alcohol ROH is tert-butyl alcohol.

5. Thieno [2,3-c] pyridine derivatives having the following formula:

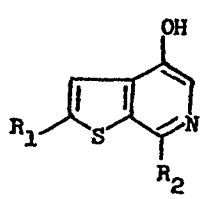 (I)

in which $R_1$ and $R_2$, which may be the same or different, represent each a hydrogen atom or an alkyl group, and their pharmaceutically acceptable acid addition salts.

6. Derivatives as claimed in claim 5, wherein the alkyl group is a $C_{1-6}$ lower alkyl group.

7. Therapeutic composition having, in particular, anti-inflammatory activities, comprising, as active ingredient, an efficient amount of at least a compound as claimed in claim 5, together with pharmaceutically acceptable carriers and excipients.

8. Therapeutic composition as claimed in claim 7, in unit dosage form, each unit dose containing 10—250 mg active ingredient.